# EUROPEAN PATENT APPLICATION

(11) **EP 3 753 533 A1**
(43) Date of publication of application: **23.12.2020**
(21) Application number: 19220078.0
(22) Date of filing: 30.12.2019
(51) Int. Cl.: A61F 2/24

(54) **VALVED STENT AND HEART VALVE WITH SAME**

(30) Priority: 17.06.2019 CN 201910522068
(71) Applicant: Fuwai Hospital Chinese Academy of Medical Sciences (CAMS), Beijing 100037 (CN); Lepu Medical Technology (Beijing) Co., Ltd., Changping Tech. Zone Beijing 102200 (CN)
(72) Inventor: ZHAO, Xuancheng, Beijing, Beijing 102200 (CN); WU, Yongjian, BEIJING, Beijing 100037 (CN); LIU, Qingrong, BEIJING, Beijing 100037 (CN); QIU, Kejin, Beijing, Beijing 102200 (CN); CHANG, Rencao, Beijing, Beijing 102200 (CN); ZHANG, Yuxin, Beijing, Beijing 102200 (CN)
(74) Representative: Lavoix

(57) **Abstract**

The invention discloses a valved stent and a heart valve with same. The valved stent has an inflow end and an outflow end, a waist portion is concavely formed near the inflow end to determine and limit a position of the valved stent, and a connecting portion is disposed at the outflow end and used for being connected with a delivery system. The heart valve comprises a valve body, a skirt edge and a valved stent, wherein the valve body is disposed on an inner side of the valved stent, the skirt edge is an elastomer, and the elastomer is coupled to the valve body and disposed on a peripheral wall of the valved stent for sealing the periphery of the valve. The heart valve provided by the invention has the function of active positioning, and can effectively prevent the positioned valved stent from shifting. The heart valve of the invention is characterized by active positioning and self-adaption of an aortic valve annulus.

## Description

### FIELD OF THE INVENTION

The invention relates to the technical field of medical instruments, in particular to a valved stent and a heart valve with same.

### BACKGROUND OF THE INVENTION

A heart valve prosthesis is an artificial device that can be implanted into the heart to replace a heart valve (an aortic valve, a tricuspid valve and a mitral valve) and enable blood to flow unidirectionally, and has the function of a natural heart valve. Heart valve prosthesis replacement is required when the heart valve has severely diseased and the valve function cannot be repaired or improved by the surgical repair or replacement of the valve.

Heart valve prostheses fall into two broad categories depending on its materials: mechanical heart valves completely made from artificial materials; and bioprosthetic heart valves made, in whole or in part, from biological tissue, typically from the materials such as porcine aortic valves and bovine pericardial valves. A basic structure of either the mechanical heart valve or the bioprosthetic heart valve comprises three portions, namely a metal valve frame, an obstructor and a sewing ring. The metal valve frame is generally made from stainless steel, titanium, cobalt-nickel alloy or other superhard metals and the like; and the sewing ring is a portion for suturing the valve prosthesis to a heart valve annulus of a human body, and is sutured from knitted materials. Polypropylene, terylene and polytetrafluoroethylene were used, and carbon fiber was also used in recent years for the sewing ring.

Transcatheter aortic valve replacement (TAVR) is a relatively advanced valve prosthesis implantation method at present, which includes retrograde (trans-arterial implantation) or anterograde (trans-apical implantation) approach. By using minimally invasive means, and utilizing vascular access, a guide wire is introduced from the femoral artery or by trans-apical approach to deliver the heart valve prosthesis into the aorta to replace the diseased valve. Compared with the surgical replacement of the heart valve, the TAVR has the advantages of no cardiopulmonary bypass, less trauma, short time and little damage to the human body.

However, the existing valve prosthesis stent has the problems of being difficult to position, inaccurate in matching between the edge of a valve leaflet and a sewing ring after positioning, and paravalvular leakage may occur. In addition, when the heart valve is closed and blood pressure of the body is maintained, shifting of the heart valve can occur, which cannot meet the requirement for normal functions of the valve.

### SUMMARY OF THE INVENTION

One of the objectives of an embodiment of the present invention is to overcome the defects in the prior art, and provide a valved stent. This heart valve has the function of active positioning and can effectively prevent the positioned valved stent from shifting.

In order to achieve the above objective, the embodiment of the present invention provides the following technical solution:
In a first aspect, a valved stent is provided, which has an inflow end and an outflow end, wherein a waist portion is concavely formed near the inflow end to determine and limit a position of the valved stent, and a connecting portion is disposed at the outflow end and used for being connected with a delivery system.

In one embodiment: the inflow end and the outflow end have a same diameter of 18 mm to 50 mm.

In one embodiment: the inflow end and the outflow end have the same diameter, a minimum diameter of the waist portion is less than the diameter of the inflow end by 1 mm to 5 mm. In one embodiment, the minimum diameter of the waist portion is less than the diameter of the inflow end by 1 mm to 3 mm.

In one embodiment: the connecting portion is a hook or a lug, and the connecting portion is hooked with the delivery system in the form of a snap fastener.

In one embodiment: the valved stent is made from a superelastic alloy and/or a shape memory alloy.

In another aspect, an embodiment of the present invention provides a heart valve. The heart valve comprises a valve body, a skirt edge and the valved stent described in the first aspect, wherein the valve body is disposed on an inner side of the valved stent, the skirt edge is an elastomer, the elastomer is coupled to the valve body and disposed on a peripheral wall of the valved stent for sealing the periphery of the valve.

In one embodiment: an inner skirt is formed by a portion of the skirt edge that is attached along an inner peripheral wall of the valved stent, an outer skirt is formed by a portion of the skirt edge that is attached along an outer peripheral wall of the valved stent after the skirt edge is folded outwardly, the inner skirt is connected with the valve body, the outer skirt is disposed to cover the waist portion, and the outer skirt abuts against a position of a valve annulus after the heart valve is positioned.

In one embodiment: the skirt edge is folded outwardly along the inflow end of the valved stent.

In one embodiment: the valve body and the skirt edge are respectively provided with corresponding suture holes for guiding sutures to suture the valve body and the skirt edge.

In one embodiment: the valve body comprises three valve leaflets, commissures of the three valve leaflets are centrally engaged to one another, and opposite ends of the commissure in each valve leaflet are fixed on the inner skirt.

In one embodiment: a plurality of fixation sites for suturing the valve body are formed on a peripheral side of the valved stent near the outflow end.

In one embodiment: the valve leaflets and the skirt edge are made from an animal pericardium or a polymeric material, preferably one or more of a bovine pericardium, a porcine pericardium, polyethylene terephthalate, polytetrafluoroethylene and polyethylene.

Compared with the prior art, the embodiments of the present invention has the following beneficial effects:
(1) According to the valved stent provided by the embodiments of the present invention, the waist portion is disposed near the inflow end, which can ensure that after the valve is implanted into the human body, the valved stent has the characteristic of active and accurate positioning, an implantation position of the valve is limited to prevent the valve from shifting, while a contact area between the valve prosthesis and an in-situ aortic valve annulus is also increased, so as to play a role in reducing paravalvular leakage.
(2) The heart valve provided by the present invention comprises a valved stent, a valve body and a skirt edge, wherein the valved stent has a waist portion, so that the heart valve has the characteristics of active positioning and self-adaption of an aortic valve annulus. In addition, the skirt edge for sealing the periphery of the valve is also disposed on the valved stent, and the skirt edge is disposed on a peripheral wall of the valved stent and connected with the valve body, which can play a role in fixing the valve body on the one hand; and on the other hand, the skirt edge is an elastomer, and can be coupled to the peripheral wall of the valved stent at any time, whether the valved stent is in a contracted state or an deployed state, which ensures contact with surrounding tissue, and further reduces paravalvular leakage.

### BRIEF DESCRIPTION OF THE DRAWINGS

In order to more clearly illustrate the technical solutions in embodiments of the present invention or in the prior art, the accompanying drawings, required in the description of the embodiments or the prior art, will now be briefly introduced. It is obvious that the drawings in the following description are only for some embodiments of the present invention, and that those ordinarily skilled in the art can obtain other drawings from these drawings without involving any inventive effort.
Fig. 1 is a schematic structural diagram of a valved stent provided by an embodiment of the present invention;
Fig. 2 is a schematic structural diagram of a heart valve provided by an embodiment of the present invention;
Fig. 3 is a schematic structural diagram of a valve leaflet in an embodiment of the present invention;
Fig. 4 is a schematic structural diagram of a skirt edge in an embodiment of the present invention;
Fig. 5 is schematic diagram of a sutured combined heart valve provided by an embodiment of the present invention;
Fig. 6 is a schematic diagram illustrating a position of a heart valve implanted into a human body provided by an embodiment of the present invention.

### Description of reference numerals:

1. valved stent; 11. inflow end; 12. outflow end; 13. waist portion; 14. connecting portion; 15. fixation sites; 2. valve body; 21. valve leaflet; 3. skirt edge; 31. inner skirt; 32. outer skirt; 4. suture holes; 5. valve annulus; and 6. coronary artery ostium.

### DETAILED DESCRIPTION OF THE INVENTION

The technical solutions of the present invention will now be described clearly and fully hereinafter with reference to the accompanying drawings. Apparently, the described embodiments are only some but not all of the embodiments of the present invention. Based on the embodiments in the present invention, all other embodiments obtained by those ordinarily skilled in the art without involving any inventive effort are within the protection scope of the present invention.

In describing the present invention, it is to be understood that the terms "center", "upper", "lower", "left", "right", "vertical", "horizontal", "inner", "outer" and the like indicate orientations or positional relationships that are based on the orientations or positional relationships shown in the drawings, are merely intended to facilitate describing the present invention and to simplify the description, are not intended to indicate or imply that the referenced device or element must have a particular orientation, be constructed and operated in a particular orientation, and thus should not be construed as limiting the invention. Furthermore, the terms "first", "second" and "third" are used for descriptive purposes only and are not to be construed as indicating or implying relative importance.

In describing the present invention, unless expressly specified and limited otherwise, it is to be understood that the terms "mounted", "connected with" and "connected" are to be interpreted broadly, for example, either fixedly connected, or detachably connected, or integrally connected; mechanically connected or electrically connected; connected with directly or indirectly through an intermediary, or communicated inside two elements. It will be understood by those of ordinary skill in the art that the specific meanings of the terms above in the present invention may be specifically understood.

Furthermore, the technical features involved in the different embodiments of the present invention described below may be combined with each other as long as they do not constitute a conflict with each other.

### Embodiment 1

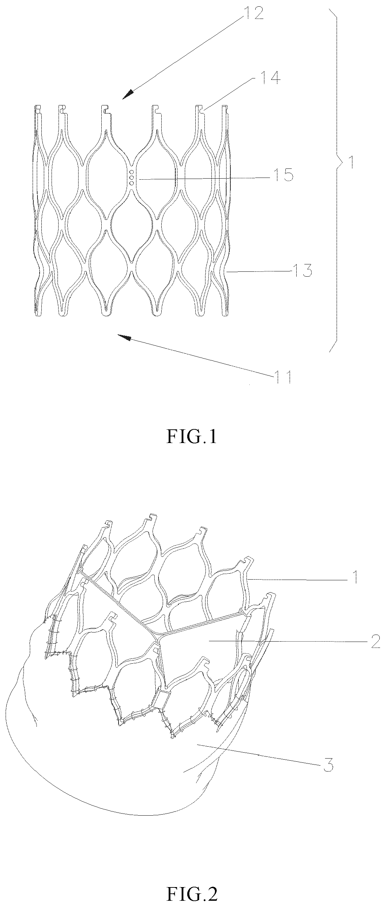

As shown in Fig. 1, the present invention provides a valved stent 1 having a cylindrical main body which can be formed by connection of a plurality of rhombic frames, can be radially contracted and deployed, and has an inflow end 11 and an outflow end 12, wherein a waist portion 13 is concavely formed near the inflow end 11 of the valved stent 1, that is, the waist portion 13 is positioned closer to the inflow end 11 than the outflow end 12, and a diameter of the waist portion 13 is slightly less than that of the inflow end 11 and the outflow end 12. When the valved stent 1 is implanted into a human body, the valved stent 1 can be actively clamped to a position of a valve annulus 5 to determine and limit a position of the valved stent 1. In addition, a connecting portion 14 is also disposed at the outflow end 12 and can be connected with an external delivery system, so that the valved stent 1 can be smoothly implanted into a human body. The delivery system described herein is primarily used for providing delivery power to the valved stent in order to deliver the valved stent 1 to a transplantation area. The delivery system in the present invention is not limited and only needs to be connectable with the valved stent 1 and capable of braking the valved stent 1, for example, the delivery system may include a handle through which the movement of the valved stent 1 is controlled. Of course during transcatheter aortic valve replacement (TAVR), a loading sheath can also be disposed at a tail end of the movable handle to receive the valved stent 1 in a contracted state, and when the valved stent 1 reaches a designated position, a force is applied to the valved stent 1 through the handle so as to release the valved stent 1 and deploy and position the valved stent 1 in the transplantation area.

According to the valved stent 1 provided by the present invention, the waist portion 13 is disposed near the inflow end 11, which can ensure that after a heart valve prosthesis is implanted into the human body, the valved stent 1 has the characteristic of active and accurate positioning, an implantation position of the heart valve prosthesis is limited to prevent the heart valve prosthesis from shifting, while a contact area between the heart valve prosthesis and an in-situ aortic valve annulus 5 is also increased due to the shape of the waist portion 13, so as to play a role in reducing paravalvular leakage. The valved stent 1 of the present invention has a positioning setting, can be used for fixing the valve and implanted together with the valve into an in-situ aortic valve which is diseased due to aortic valve stenosis or aortic regurgitation/insufficiency, and has the characteristic of active positioning and self-adaption of the aortic valve annulus. Likewise, the valved stent 1 can also be used for implantation in a mitral valve that is diseased due to mitral valve stenosis or mitral regurgitation, and has the characteristic of active positioning and self-adaption of a mitral valve annulus. Or likewise, the valved stent 1 can also be used for implantation in a tricuspid valve that is diseased due to tricuspid valve stenosis or tricuspid regurgitation, and has the characteristic of active positioning and self-adaption of a tricuspid valve annulus.

In this embodiment, preferably the inflow end 11 and the outflow end 12 have the same diameter of 18 mm to 50 mm, wherein a minimum diameter of the waist portion 13 is less than the diameter of the inflow end 11 or the outflow end 12 by 1 mm to 5 mm, preferably 1 mm to 3 mm, which can ensure that the valved stent 1 has proper supporting rigidity, and the valved stent 1 can be smoothly contracted, while the valved stent 1 can also be effectively prevented from shifting. Among other things, the valved stent 1 of the present invention may be made from a superelastic alloy and/or a shape memory alloy, so that the heart valve prosthesis provided with the valved stent 1 may be radially contracted and deployed and implanted to a desired position by transcatheter means, for example, implanted to the position of the aortic valve. Of course the delivery system required in an implantation process can be connected with the valved stent 1 through the connecting portion 14, and the connecting portion 14 may be a hook or a lug. When the connecting portion 14 is the hook, the lug corresponding to the hook may be disposed on the delivery system; and when the connecting portion is the lug, the hook corresponding to the lug may be disposed on the delivery system, so as to achieve hooking in the form of a snap fastener, and facilitate delivery and recovery of the delivery system.

### Embodiment 2

As shown in Fig. 2, the present invention provides a heart valve having a positioning setting. The heart valve comprises a valve body 2, a skirt edge 3 and the valved stent 1 described above, wherein the valve body 2 is disposed on an inner side of the valved stent 1, the skirt edge 3 is an elastomer, the elastomer is coupled to the valve body 2 and disposed on a peripheral wall of the valved stent 1 for sealing the periphery of the valve.

The heart valve provided by the present invention comprises a valved stent 1 with a waist portion 13, so that the heart valve has the characteristic of active positioning and self-adaption of an aortic valve annulus 5. In addition, a skirt edge for sealing the periphery of the valve is also disposed on the valved stent 1, and the skirt edge is disposed on the peripheral wall of the valved stent 1 and connected with the valve body 2, which can play a role in fixing the valve body 2 on the one hand; and on the other hand, the skirt edge 3 is an elastomer, and can be positioned on the peripheral wall of the valved stent 1 at any time, whether the valved stent 1 is in a contracted state or an deployed state, which ensures contact with surrounding tissue, and further reduces paravalvular leakage.

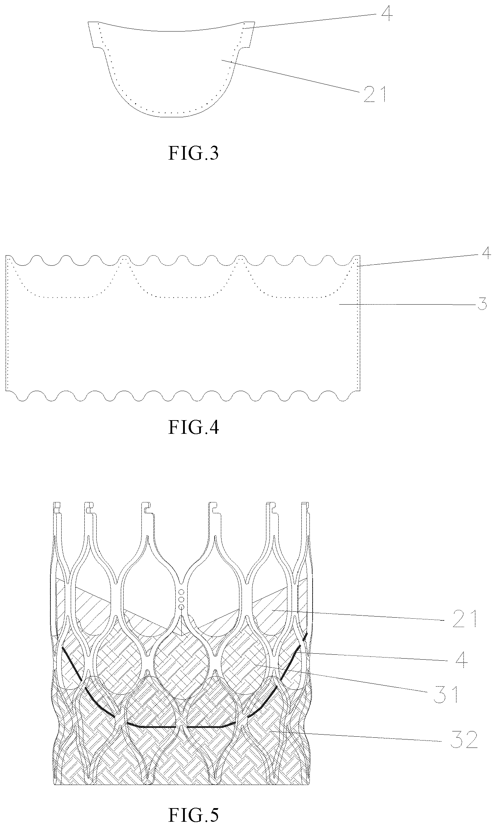

Specifically, as shown in Fig. 2, the valve body 2 comprises three valve leaflets 21, and commissures of the three valve leaflets 21 are centrally engaged to one another. As shown in Fig. 3, edges of opposite ends of the commissure in each valve leaflet 21 are respectively provided with uniform suture holes 4. The purpose of the suture holes 4 is to ensure that during the process of suturing and combining the valve leaflets 21 and the skirt edge 3, the process is stable, and suture points are uniform, without being influenced by manual skills of workers, so that the stability of hemodynamic characteristics of the heart valve can be further ensured, and materials of the valve leaflets 21 and the skirt edge 3 are protected from being damaged by a suture needle simultaneously.

As shown in Fig. 5, in this embodiment, the skirt edge 3 comprises an inner skirt 31 and an outer skirt 32 which are integrally formed, wherein an inner skirt 31 is formed by a portion of the skirt edge 3 that is attached along an inner peripheral wall of the valved stent 1, and an outer skirt 32 is formed by a portion of the skirt edge 3 that is attached on an outer peripheral wall of the valved stent 1 after the skirt edge 3 is folded outwardly along the inflow end of the valved stent 1. As shown in Fig. 4, the inner skirt 31 is partially provided with suture holes 4, and the number of the suture holes 4 corresponds to suture holes 4 in the edge of each valve leaflet 21 in a one-to-one manner, wherein the process of suturing the valve leaflets 21 and the skirt edge 3 on the valved stent 1 comprises the following steps: firstly combining the valve leaflets 21 and the skirt edge 3 together along the suture holes 4 by a way of suturing, then suturing a portion of the inner skirt 31 on an inner wall of the valved stent 1, turning a portion of the outer skirt 32 outwards and suturing the outer skirt 32 on an outer wall of the valved stent 1. During suturing, edges of the inner skirt 31 and the outer skirt 32 are respectively sutured on edges of the rhombic frames of the valved stent 1. As shown in Fig. 1, fixation sites 15 may be formed on the rhombic frames near the outflow end 11, and are distributed along an axial direction of the valved stent 1 with three fixation sites 15 as a group. As shown in Fig. 2, each group of fixation sites 15 is disposed corresponding to the valve leaflets 21 of the valve body 2 to stably suture the valve leaflets 21 on the valved stent 1 in a wrapping manner, while the outer skirt 32 can preferably cover the waist portion 13 of the valved stent 1. After the heart valve is positioned, the outer skirt 32 abuts against the position of a valve annulus 5, namely between the metal valved stent 1 and corresponding human tissue, so that on the one hand, the friction of metal on the human tissue can be reduced, and on the other hand, a sealing effect can be achieved to reduce paravalvular leakage. The outer skirt 32 may be made from animal pericardial tissue, such as a bovine pericardium, a porcine pericardium, an equine pericardium and the like, or artificial synthetic materials, such as PET, PTFE, PE and the like.

Fig. 5 is a schematic diagram of a sutured combined heart valve of the present invention. The inner skirt 31 and the outer skirt 32 are respectively attached to the inner side and the outer side of the valved stent 1.

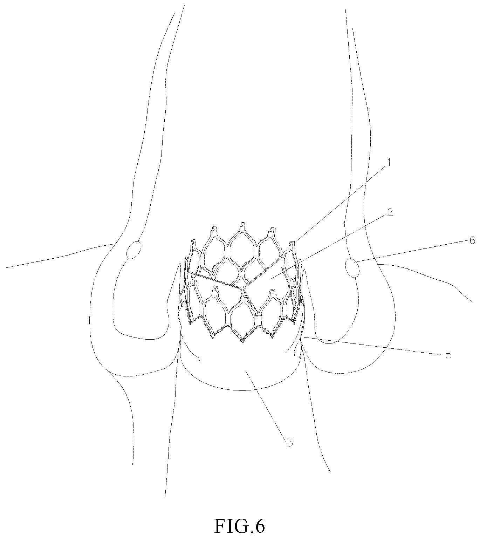

The position of the heart valve according to the example of the present invention implanted into the human body is shown in Fig. 6. As the valved stent 1 of the present invention may be made from the superelastic alloy and/or the shape memory alloy, the heart valve of the present invention may be implanted to the desired position by transcatheter means, for example, implanted to the position of the aortic valve. As shown in Fig. 6, after the heart valve is implanted, the waist portion 13 of the valved stent 1 is disposed to self-adapt to the position of the valve annulus 5 of an original aortic valve to achieve the effect of automatic positioning, so that vertical shifting of the heart valve at the position of the original aortic valve is limited, and the fixing capacity of the heart valve is improved; meanwhile, the outer skirt 32 of the heart valve closely attached to the position of the valve annulus 5 to play a role of sealing, so as to reduce or prevent paravalvular leakage. Furthermore, in view of the fact that the appropriate position for implantation of the valve prosthesis into aorta is generally 6-12 mm below the valve annulus, and the distance from coronary artery ostium to the bottom of aortic sinus is generally required to be greater than 15 mm in TAVR, in this embodiment, the valved stent 1 having a height of less than 25 mm, preferably 16-25 mm, is selected so as to ensure that the heart valve provided by the present invention is implanted behind the position of the original aortic valve, and keeps away from the position of the coronary artery ostium 6, so that the obstruction of the coronary artery ostium 6 cannot be caused, and other coronary artery surgeries carried out on the patient in future cannot be influenced.

The above description is only specific embodiments of the present invention, but the protection scope of the present invention is not limited thereto. Any variations or substitutions, readily occur to those skilled in the art within the technical scope disclosed by the present invention, are intended to be within the protection scope of the present invention. Therefore, the protection scope of the present invention should be determined with reference to the appended claims.

## Claims

1. A valved stent **characterized by** having an inflow end and an outflow end, a waist portion being concavely formed near the inflow end to determine and limit a position of the valved stent, a connecting portion being disposed at the outflow end and used for being connected with a delivery system.

2. The valved stent according to claim 1, **characterized in that** the inflow end and the outflow end have a same diameter of 18 mm to 50 mm.

3. The valved stent according to claims 1 or 2, **characterized in that** the inflow end and the outflow end have the same diameter, a minimum diameter of the waist portion is less than the diameter of the inflow end by 1 mm to 5 mm; further the minimum diameter of the waist portion is less than the diameter of the inflow end by 1 mm to 3 mm.

4. The valved stent according to any one of claims 1-3, **characterized in that** the connecting portion is a hook or a lug, and the connecting portion is hooked with the delivery system in the form of a snap fastener.

5. The valved stent according to any one of claims 1-4, **characterized in that** the valved stent is made from a superelastic alloy and/or a shape memory alloy.

6. A heart valve **characterized by** comprising a valve body, a skirt edge and a valved stent according to any one of claims 1-5, the valve body being disposed on an inner side of the valved stent, the skirt edge being an elastomer, the elastomer being coupled to the valve body and disposed on a peripheral wall of the valved stent for sealing a periphery of the valve.

7. The heart valve according to claim 6, **characterized in that** an inner skirt is formed by a portion of the skirt edge that is attached along an inner peripheral wall of the valved stent, an outer skirt is formed by a portion of the skirt edge that is attached along an outer peripheral wall of the valved stent after the skirt edge is folded outwardly, the inner skirt is connected with the valve body, the outer skirt is disposed to cover the waist portion, and the outer skirt abuts against a position of a valve annulus after the heart valve is positioned.

8. The heart valve according to claims 6 or 7, **characterized in that** the skirt edge is folded outwardly along the inflow end of the valved stent.

9. The heart valve according to any one of claims 6-8, **characterized in that** the valve body and the skirt edge are respectively provided with corresponding suture holes for guiding sutures to suture the valve body and the skirt edge.

10. The heart valve according to any one of claims 6-9, **characterized in that** the valve body comprises three valve leaflets, commissures of the three valve leaflets are centrally engaged to one another, and opposite ends of the commissure in each valve leaflet are fixed on the inner skirt.

11. The heart valve according to any one of claims 6-10, **characterized in that** a plurality of fixation sites for suturing the valve body are formed on a peripheral side of the valved stent near the outflow end.

12. The heart valve according to claim 10, **characterized in that** the valve leaflets and the skirt edge are made from an animal pericardium or a polymeric material, preferably one or more of a bovine pericardium, a porcine pericardium, polyethylene terephthalate, polytetrafluoroethylene and polyethylene.
